# EUROPEAN PATENT APPLICATION

(11) **EP 3 034 131 A1**
(43) Date of publication of application: **22.06.2016**
(21) Application number: 15201283.7
(22) Date of filing: 18.12.2015
(51) Int. Cl.: A61Q 15/00, A61K 8/02, A61K 8/06, A61K 8/04, A61K 8/64

(54) **ANTIPERSPIRANT COMPOSITION**

(30) Priority: 19.12.2014 US 201414577136
(71) Applicant: Johnson & Johnson Consumer Inc., Skillman, NJ 08558 (US)
(72) Inventor: ANCONI, Glasiela Lemos, 12245-500 Sao Jose dos Campos (BR); DE CASTRO MONTEIRO LOFFREDO, Luciana, 12242-431 Sao Jose dos Campos (BR); PASSERO, Alan, 12305-650 Jacarei (BR); SHERGUE, Ellen Muniz, 02436-000 Sao Paulo (BR); MACEDO, Filomena A., Hillsborough, NJ New Jersey 08844-2250 (US)
(74) Representative: Carpmaels & Ransford LLP

(57) **Abstract**

The present invention relates to a topical antiperspirant composition comprising a cosmetically acceptable topical carrier and an anticholinergic agent selected from the group consisting of acetyl octapeptide-3 and dipeptide diaminobutyroyl benzylamide diacetate in an amount effective to inhibit or prevent perspiration, wherein said composition is substantially free of aluminum salts.

## Description

### Field of the invention

This invention relates to a topical antiperspirant composition containing an anticholinergic agent as the active antiperspirant compound that is substantially free of aluminum salts.

### Background of the Invention

Antiperspirant and deodorant compositions are widely employed throughout many parts of the world in order to control localized perspiration and odor. Wet patches such as those under the arms are considered to be unsightly. Additionally, body odor can interfere with normal relationships and cause social exclusion. These issues take on a greater significance particularly in regions that tend to have high humidity and warmer climates. Besides the underarm area, other body areas including the feet may benefit from the use of antiperspirants and deodorants.

Antiperspirants act by stopping or reducing the activity of the sweat glands. Today, antiperspirants are typically sold in a rollerball applicator or solid stick form and include an aluminum-based compound, wax, liquid emollients and natural scent enhancers. Antiperspirants currently marketed commonly employ an astringent metal salt, such as aluminum or aluminum/zirconium salt. The key to the sweat-blocking activity rests purely in the active aluminum-based compound that, in a typical commercial antiperspirant, makes up from about 10 to 25 percent of the ingredients. Aluminum ions, from aluminum salts such as aluminum chlorohydrate and aluminum chloride, are drawn into the cells that line the human body's eccrine gland ducts. The eccrine glands are responsible for producing the majority of the body's sweat and are located in the armpits. The aluminum ions are taken into the cells that line the eccrine-gland ducts at the opening of the epidermis, the top layer of the skin. When the aluminum ions are drawn into the cells, water passes in with them. As more water flows in, the cells begin to swell, squeezing the ducts closed. Consequently, sweat is directly blocked from being excreted through the skin. Once the eccrine ducts have been closed, the other odor-reducing/masking ingredients provide a thin coating to the skin's top layer. The gland ducts remain closed until the water content outside and inside the gland cells reaches equilibrium at which time the cell content begins to pass back out through osmosis.

Deodorants on the other hand block the odor of sweat. Deodorant agents do this by limiting bacterial proliferation, blocking sweat diffusion, absorbing malodors and enzymatic inhibition. Commercial products may be marketed as a deodorant, an antiperspirant or both.

U.S. Patent Publication No. 2011/0020415 relates to a composition for the treatment of hyperhidrosis. It contains an antiperspirant, at least one muscle relaxing agent, at least one peptide, and at least one botanical agent. The antiperspirant is preferably aluminum tertacholrohydrex glycine, and the peptide may be for example dipeptide diaminobutyroyl benzylamide diacetate, argireline, SYN-AKE, SNAP 8, or the like.

Recently, the use of aluminum in deodorants and antiperspirants has become controversial due to concern that aluminum can be absorbed by the body and enter into the bloodstream. Additionally, antiperspirant aerosols containing aluminum allow for inhalation during manufacture and application.

The present invention provides a novel topical antiperspirant product that does not require aluminum salts. These antiperspirant compositions are substantially free, preferably 100% free, of aluminum salts.

### Summary of the Invention

The present invention provides a topical antiperspirant composition comprising a cosmetically acceptable topical carrier and an anticholinergic agent selected from the group consisting of acetyl octapeptide-3 and dipeptide diaminobutyroyl benzylamide diacetate in an amount effective to inhibit or prevent perspiration, wherein said composition is substantially free of aluminum salts.

As used herein, the term "comprising" encompasses "including" as well as "consisting" and "consisting essentially of" e.g. a composition "comprising" X may consist exclusively of X or may include an additional component, e.g. X + Y.

### Detailed Description of the Invention

The cosmetic antiperspirant composition of the invention is adapted to be applied topically to a user's skin, typically in the underarm area.

The composition is substantially free of aluminum salts. In particular, the composition is substantially free of aluminum chlorohydrate and/or its derivatives. The formulations may occasionally contain aluminum residue. This is related to traces or extremely low amounts of aluminum element as a residue in raw materials used as ingredients of the composition. This is not an aluminum salt.

As used herein, "substantially free of" an ingredient means containing less than about 50 ppm in final formulation.

In one embodiment, the composition of the invention is totally free of aluminum salts.

### Anti-cholinergic Agents

The anti-cholinergic agent in the composition is acetyl octapeptide-3 (for example SNAP 8 commercially available from Lipotec SA) or dipeptide diaminobutyroyl benzylamide diacetate (for example SYN-AKE commercially available from Pentapharm Ltd.).

While not wishing to be being bound by theory, it is believed that dipeptide diaminobutyroyl benzylamide diacetate acts by blocking the acetylcholine receptor of sweat glands, while acetyl octapeptide-3 acts by preventing the release of acetylcholine from sympathetic neurons.

The anticholinergic agent is present in the composition in an amount effective to inhibit or substantially reduce perspiration, for example in the underarm area of a human user following topical application to the same. The composition will generally contain about 0.01 to about 30.0 w/w% of the anticholinergic agent.

In one embodiment the composition contains about 0.1 to about 20.0 w/w%, or about 0.5 to about 15.0 w/w%, of acetyl octapeptide-3.

In another embodiment the composition contains about 0.05 to about 6.0 w/w%, or about 0.1 to about 4.0 w/w%, of dipeptide diaminobutyroyl benzylamide diacetate.

The composition may optionally also comprise one or more other anticholinergic agents, including for example biopeptides such as pentapeptide, hexapeptide, or octapeptide, botulinum toxin (botox), acetyl hexapeptide-8, trihexyphenidyl (artane), benztropine mesylate (cogentin), biperiden, procyclidine, 2,5 antihistamines (orphenadrine), atropine, flavoxate (urispas), oxybutynin (ditropan, oxytrol), scopolamine, hyoscyamine (levsinex), tolterodine (detrol), belladonna alkaloids , fesoterodine (toviaz), solifenacin (vesicare), darifenacin (enablex), propantheline (pro-banthine).

### Water Absorbing Agent

The composition may optionally comprise a water absorbing agent. Such water absorbing agents are known for cosmetic applications and are usually porous ingredients, composed of micro channels resulting in a high absorption capacity and the ability to absorb a different variety of materials. Such compounds may have affinity to hydrophilic or lipophilic molecules. In some cases, it is preferred to combine multiple water absorbing agents in order to maximize the absorption capabilities.

Suitable water absorbers include silicas, cotton powder, bamboo silk and zeolites.

In one embodiment, the composition comprises Bambusa Arundinacea Stem Powder (for example BAMBOO SILK PW available from Polytechno Indústrias Químicas Ltda/lon Química), silicas (for example MSS 500/3H available from Kobo and/or SPHERON L-1500 available from Presperse).

The cosmetic antiperspirant compositions of the present invention will generally contain from about 0.01 to about 20.0 w/w% of a water absorbing agent in the final composition. In a preferred embodiment, the composition will contain about 0.1 to about 10.0 w/w% and in a more preferred embodiment, the cosmetic antiperspirant composition will contain about 0.5 to about 6.0 w/w% of a water absorbing agent.

The composition may comprise other ingredients known in the antiperspirant, deodorant, or cosmetic art. In most embodiments, the antiperspirant will include other functional ingredients, which provide benefits to the user's body. Such materials are in general well-known to those persons of ordinary skill in the relevant personal care composition art, and may include moisturizing agents, anti-bacterial agents, deodorants and perfumes.

Other ingredients may include film formers. For example, water-insoluble films of polymers create occlusion on the axillary skin, thereby reducing the underarm wetness. Such films are a barrier to the passage of sweat. Polymers used should be occlusive, insoluble in water, and high in degree of adhesion, such as Acrylates/Octylacrylamide Copolymer.

The composition may also include ingredients known to have antibacterial and deodorant properties. These substances may include, for example, 1,6-di-(4-chlorophenylbiguanido) hexane (Chlorhexidin), 3,4,4'-trichlorocarbanilide, quaternary ammonium compounds, oil of cloves, mint oil, thyme oil, triethyl citrate, farnesol (3,7,11-trimethyl-2,6,10-dodecatrien-1-ol), ethylhexyl glycerol ether, or polyglyceryl-3 caprylate. In particular, polyglyceryl 3 caprylate (TEGO® Cosmo P 813, available from Degussa/Evonik), a vegetable sourced ester, with lipophilic character, may be used to reduce odor-causing bacteria on the skin at very low concentrations.

The composition may also comprise linear chain alcohols, amines, ether-alcohols, cetyl alcohol, stearyl alcohol and cetearyl alcohol, classified by CTFA as "emulsion stabilizers" agents.

Perfumes and fragrances may be included in the antiperspirant composition. Perfumes give rise to an olfactory response in the form of a fragrance, and in addition may contribute to mask body malodor.

Preservatives are required to prevent product damage caused by bacteria, yeast or mold, and to protect the product from inadvertent contamination by the consumer during use. Useful preservatives include sodium benzoate, ethylhexylglycerin and caprylyl glycol, benzyl alcohol, methyl paraben, propyl paraben, DMDM hydantoin, methylchloroisothiaoline, methylisothiazolinone, imidazolidinyl urea phenoxyethanol, sodium benzoate and benzoic acid. EDTA and salts thereof are often used to further enhance preservation.

Antioxidants should be added to prevent formula from oxidation process. Useful antioxidants include ascorbic acid, tocopheryl acetate and polypnehols. In the present invention, anti-oxidants such as tocopheryl acetate (stabilized vitamin E) may be used as anti-oxidant agent.

Humectants and moisturizing agents may be included in the composition. Suitable ingredients include hydrophobic agents, hydrophilic agents and combinations thereof. Examples of moisturizing agents are allantoin, glycerol, polyglycerylmethacrylate, panthenol, polyols, ceramide, borage oil (linoleic acid), hyaluronic acid, sodium peroxylinecarbolic acid (sodium PCA), wheat protein (e.g., laurdimonium hydroxypropyl hydrolyzed wheat protein), hair keratin amino acids, panthenol; primrose oil; GLA 3 and other fish oils that may include, for example, the omega-3 and omega-6 oils and/or linoleic acid; and flax seed oil, and mixtures thereof. Other moisturizing agents can also be used.

Additionally, skin conditioners may be added to prevent or calm skin irritation. For example, vegetable extracts may be added. Cotton milk (commercially available from Symrise) may provide nourishing, calming and softening properties to skin. In one embodiment, the composition of the invention contains about 34 to about 40 weight percent milk proteins.

Pigments and colorants may be included in the antiperspirant composition of the present invention.

Emollients, such as isopropylmyristate, mineral oils and vegetable oils in general, which give rise to a tactile response in the form of an increase in skin lubricity may be included for a more esthetically pleasing product. The composition may contain emollients in any desired amount to achieve a desired emollient effect.

Non-volatile emollients are preferable in the present invention. Classes of non-volatile emollients include non-silicone and silicone emollients. Non-volatile, non-silicone emollients include C₁₂₋₁₅ alkyl benzoate. The non-volatile silicone material can be a polyethersiloxane, polyalkyarylsiloxane or polyethersiloxane copolymer. An illustrative non-volatile silicone material in the present invention is phenyl trimethicone. Non-limiting examples of emollients can be found in U.S. Pat. No. 6,007,799. Examples include, but are not limited to, PPG-14 butyl ether, PPG-15 stearyl ether, PPG-3 myristyl ether, stearyl alcohol, stearic acid, glyceryl monoricinoleate, isobutyl palmitate, glyceryl monostearate, isocetyl stearate, sulphated tallow, oleyl alcohol, propylene glycol, isopropyl laurate, mink oil, sorbitan stearate, cetyl alcohol, hydrogenated castor oil, stearyl stearate, hydrogenated soy glycerides, isopropyl isostearate, hexyl laurate, dimethyl brassylate, decyl oleate, diisopropyl adipate, n-dibutyl sebacate, diisopropyl sebacate, 2-ethyl hexyl palmitate, isononyl isononanoate, isodecyl isononanoate, isotridecyl isononanoate, 2-ethyl hexyl palmitate, 2-ethyl hexyl stearate, Di-(2-ethyl hexyl) adipate), Di-(2-ethyl hexyl) succinate, isopropyl myristate, isopropyl palmitate, isopropyl stearate, octacosanol, butyl stearate, glyceryl monostearate, polyethylene glycols, oleic acid, triethylene glycol, lanolin, castor oil, acetylated lanolin alcohols, acetylated lanolin, petrolatum, isopropyl ester of lanolin, fatty acids, mineral oils, butyl myristate, isostearic acid, palmitic acid, PEG-23 oleyl ether, olelyl oleate, isopropyl linoleate, cetyl lactate, lauryl lactate, myristyl lactate, quaternised hydroxy alkyl, aminogluconate, vegetable oils, isodecyl oleate, isostearyl neopentanoate, myristyl myristate, oleyl ethoxy myristate, diglycol stearate, ethylene glycol monostearate, myristyl stearate, isopropyl lanolate, paraffin waxes, glycyrrhizic acid, hydrocyethyl stearate amide.

In one embodiment, the emollient is selected from linear silicones, cyclic silicones, hydrocarbons, polyhydroxy alcohols having more than 3 carbon atoms, liquid or solid polyalkyleneglycol ethers containing a polypropylene glycol (PPG) moiety and terminating in an alkyl ether, and combinations thereof. In another embodiment, the emollient is a volatile silicone having a flash point of 100° C. or less, such as cyclomethicone or trisiloxane. In another embodiment, the emollient is a nonvolatile silicone, such as dimethiconol or a longer chain dimethicone.

Surfactants and emulsifiers may be used in the antiperspirant compositions of the invention. Typical surfactants are disclosed in U.S. 2003/0007939A1. Emulsifiers useful in the composition include nonionic, anionic, cationic, amphoteric or zwitterionic and blends thereof. Suitable emulsifiers are disclosed in U.S. Patent No. 3,755,560 and U.S. Patent No. 4,421,769. Examples are polyethylene glycol 20, sorbitan monolaurate (Polysorbate 20), polyethylene glycol 20 stearyl ether (Brij 78, Steareth 20), polyethylene glycol ether of lauryl alcohol (Laureth 23), polysorbate 80 (Tween 80), and lecithin.

Cellulosic gums also can be used as additives in the compositions of this invention. Preferred cellulosic gums include water-soluble hydroxyalkylcellulose polymers such as hydroxymethylcellulose, hydroxyethylcellulose and hydroxypropylcellulose. Other thickening agents that may be used include acacia, agar, alginate, carrageenan, gum tragacanth, xanthan gum, collagen, carboxypolymethylene, glyceryl monostearate, polyvinylpyrrolidone and polyacrylamide.

The composition may be in the form of a solid or gel stick, cream, lotion, spray, squeeze product, or the like, as known in the art.

Accordingly, the anticholinergic agent and other active agents may be contained in a vehicle, as known in the art. The major types of antiperspirant vehicles most frequently fall into the following categories: (a) solutions; (b) emulsions, both oil-in-water and water-in-oil; and including lotions and creams; (c) suspensions; (d) gels; and (e) solids and semi-solids including stick products. Antiperspirant products in some vehicles, including liquids, gels, suspensions and emulsions, can be provided for application via roll-on applicator, as known in the art.

Examples of solvents, in addition to water, that are frequently used in personal care compositions are polypropylene glycol, polyethylene glycol, ethanol, glycerol, ethylene glycol, 1,2,4-butanetriol, 1,2,6-hexanetriol, ethanol, isopropanol, butanetriol, sorbitol esters, butanediol, butylene glycol, hexylene glycol, methylpropanediol, pyrrolidone, N-methylpyrrolidone, dimethyl sulfoxide, dimethyl sulfone and similar solvents and mixtures thereof.

The composition of the invention may be a squeeze product, including hydro-alcoholic squeeze products.

The antiperspirant composition can be in gel form. A "gel" in accordance with the present invention is a colloid in which the disperse phase has combined with the continuous phase to produce a viscous, jelly-like product. Gels in accordance with the present invention can be aqueous or nonaqueous. The gels will typically comprise a vehicle comprising a gelling agent such as described above. The vehicle of the gels will also typically comprise a solvent. A silicone surfactant may also be included required to achieve stability.

Emulsions, such as lotions and creams, of the oil-in-water type and water-in-oil type are well-known in the cosmetic art and are useful in the subject invention. Triphase emulstions such as water-in-oil-in-water type may also be used. Oils useful in both types of emulsions, and also for solvents in solvent-based vehicles in general, include hydrocarbon oils and waxes (e.g., petrolatum, mineral oil, micro-crystalline waxes, polyalkenes, paraffins, cerasin, ozokerite, polyethylene, perhydrosqualene, polyalphaolefins, hydrogenated polyisobutenes and combinations thereof). Preferred are fatty acid derivatives, cholesterol, cholesterol derivatives, diglycerides and triglycerides (e.g., castor oil, soy bean oil, derivatized soybean oils such as maleated soy bean oil, safflower oil, cotton seed oil, corn oil, walnut oil, peanut oil, olive oil, cod liver oil, almond oil, avocado oil, palm oil, sesame oil, vegetable oils and vegetable oil derivatives, sunflower seed oil, coconut oil and derivatized coconut oil, cottonseed oil and derivatized cottonseed oil, jojoba oil, cocoa butter and combinations thereof, as well as any of the aforementioned oils that have been partially or fully hydrogenated), acetoglyceride esters (e.g., acetylated monoglycerides), alkyl esters, alkenyl esters (e.g., oleyl myristate, oleyl stearate, oleyl oleate, and combinations thereof), lanolin and its derivatives (e.g., lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol ricinoleate, hydroxylated lanolin, hydrogenated lanolin and combinations thereof), wax esters (e.g., beeswax and beeswax derivatives, spermaceti, myristyl myristate, stearyl stearate and combinations thereof), sterols and phospholipids, and combinations thereof. Examples of alkyl esters include isopropyl esters of fatty acids and long chain esters of long chain fatty acids, e.g., SEFA (sucrose esters of fatty acids), pentaerythritol esters, aromatic mono, di or triesters, cetyl ricinoleate, isopropyl palmitate, isopropyl myristate, cetyl ricinoleate and stearyl ricinoleate. Other examples include hexyl laurate, isohexyl laurate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and combinations thereof. Still other suitable oils include milk triglycerides (e.g., hydroxylated milk glyceride) and polyol fatty acid polyesters. Also useful are vegetable waxes such as carnauba and candelilla waxes; sterols such as cholesterol, cholesterol fatty acid esters; and phospholipids such as lecithin and derivatives, sphingo lipids, ceramides, glycosphingo lipids, and combinations thereof.

The water-based roll-on is usually an oil-in-water emulsion rather than a water-in-oil system due to the poorer efficiency of the latter. The oil-in-water emulsion presents the active ingredient in a readily accessible solution form in the external phase. Since the active ingredient ends up in the dissolved state, the formulator can use either a liquid or solid antiperspirant active.

The composition of the invention may also be a clear water-in-oil roll-on. These compositions are relatively new on the market. They demonstrate superior aesthetics and leave no residue or deposit on the skin after application. Clarity is achieved simply by following the room temperature order of addition specified.

The composition may alternatively be an aerosol. Sprays or aerosols have the advantage of eliminating direct contact with hands for application. The propellant gas may be, for example, a hydrocarbon (propane, isobutene), a chlorofluorocarbon, carbon dioxide or nitrous oxide.

The composition of the invention may be a suspension.

In one embodiment, the composition is a lotion or a cream.

In another embodiment, the composition is a solid or semi-solid or stick, and comprises an effective amount of anticholinergic agent and a solidifying agent, wherein the composition is in the form of a solid or semi-solid at ambient temperature (e.g., 25° C. and below). Accordingly, the composition may comprise for example mixtures of waxes and oils and solutions based on aqueous, propylene glycol and/or alcohol mixtures solidified for example by sodium stearate. Preferably, the solidifying agent is selected from the group consisting of a wax and sodium stearate.

Solid compositions of the invention may also be finely divided and used in the form of powders.

Almost all antiperspirant solids employ stearyl alcohol as the gelling agent. Emollients are often added to impart a softer feel and to add glideability. Most solids also contain talc and/or silica. Silica is an effective suspending agent that helps to keep the active ingredient homogeneously suspended throughout the stick. Miscellaneous ingredients often include colours, titanium dioxide (opacifying agent) and allantoin (soothing agent).

Sticks and solids are complex chemical systems requiring a balance of ingredients designed to make the most of several performance factors including payout, slip or lubricity, chemical stability, softening temperature, and efficacy.

The composition of the invention may also be a foam. Accordingly, the liquid vehicles described above may contain dispersions of gas in the liquid phase. The gas globules may be of any size, from colloidal to macroscopic, as in soap bubbles. Typical liquid foams are those used in shaving creams, etc.

### EXAMPLES

### Example 1

The following is composition is a hydro-alcoholic squeeze spray formulation of the present invention.

**TABLE 1**

| INGREDIENT | FUNCTION | % w/w |
|---|---|---|
| A: SD Alcohol 40 | Solvent | 38.00 |
| B: Propylene glycol | Humectant | 3.50 |
| C: Deionized water | Solvent | 48.00 |
| D: Absorbents | Absorbents | 6.00 |
| E: Fragrance | Perfume | 0.50 |
| F: Acetyl octapeptide-3 or dipeptide diaminobutyroyl benzylamide diacetate | Antiperspirant | 4.00 |

Procedure
1. Mix A and C using overhead stirrer
2. Add B. Mix for 10 min
3. Add D, E and F. When homogeneous, pour into suitable containers

### Example 2

The following product is a water-in-oil emulsion according to the invention in which the refractive indices of the continuous and dispersed phases are identically matched. A silicone surfactant is used to achieve a stabile emulsion.

**TABLE 2**

| INGREDIENT | FUNCTION | % w/w |
|---|---|---|
| A: Cyclomethicone (and) Dimethicone Copolyol | Solvent | 9.70 |
| B: Cyclomethicone | Solvent | 3.40 |
| C: Dimethicone, 50 cst | Skin Conditioning | 3.88 |
| D: Isostearyl Palmitate | Skin Conditioning | 2.42 |
| E: Dipropylene Glycol | Solvent | 19.51 |
| F: Deionized Water | Solvent | 50.69 |
| G: Absorbents | Absorbents | 6.00 |
| H: Fragrance | Perfume | 0.40 |
| I: Acetyl octapeptide-3 or dipeptide diaminobutyroyl benzylamide diacetate | Antiperspirant Agent | 4.00 |

Procedure
1. Combine A, B, C, D and H and mix until uniform
2. In a separate vessel combine E, F and G and mix until uniform
3. Match the refractive indexes of phases 1 and 2
4. Slowly add the aqueous phase from Step 2 to the oil phase from Step 1 and homogenize at slow speed. The viscosity will slowly increase.
5. Add I and mix until uniform.
6. Homogenize vigorously (5000 rpm) until a firm gel is obtained (typically from 1 to 5 min)
7. Fill into airtight containers

### Example 3

The following composition is an emulsion roll-on formulation according to the invention, which exhibits excellent physical stability and application properties.

**TABLE 3**

| INGREDIENT | FUNCTION | % w/w |
|---|---|---|
| A: Steareth-21 | Surfactant | 2.00 |
| B: Steareth-2 | Surfactant | 2.00 |
| C: Steareth-5 Stearate | Skin Conditioning | 1.00 |
| D: Clyclomethicone (and) PPG-15 Stearyl Ether | Skin Conditioning | 5.00 |
| E: Deionized Water | Solvent | 79.20 |
| F:Absorbents | Absorbents | 6.00 |
| G: Fragrance | Perfume | 0.80 |
| H: Acetyl octapeptide-3 or dipeptide diaminobutyroyl benzylamide diacetate | Antiperspirant Agent | 4.00 |

Procedure
1. Combine A, B, C and D and heat to 700° C
2. Heat E separately at 700° C
3. Add A, B, C and D to E with agitation
4. Homogenize the mixture for 1-3 min
5. Cool to 35° C with continuous agitation
6. Add F to emulsion slowly with agitation
7. Add H and G with agitation
8. Homogenize the mixture again for 1-3 min
9. Fill into suitable containers

### Example 4

This composition according to the invention provides superior aesthetics and leaves no residue or deposit on the user's skin after application. It is a clear antiperspirant product made with the following ingredients using the order of addition below at room temperature (20 -22° C).

**TABLE 4**

| INGREDIENT | FUNCTION | % w/w |
|---|---|---|
| A: Deionized Water | Solvent | 8.75 |
| B: Dipropylene Glycol | Solvent | 3.00 |
| C: PEG-7 Glyceryl Cocoate | Skin Conditioning | 18.20 |
| D: Cyclomethicone (and) Dimethicone Copolyol | Skin Conditioning | 41.50 |
| E: Cetearyl Octanoate | Surfactant | 3.20 |
| F: Polysorbate 20 | Surfactant | 1.00 |
| G: Deionized Water | Solvent | 12.60 |
| H: Isopropyl Myristate | Skin Conditioning | 1.00 |
| I: Absorbents | Absorbents | 6.00 |
| J: Fragrance | Perfume | 0.75 |
| K: Acetyl octapeptide-3 or dipeptide diaminobutyroyl benzylamide diacetate | Antiperspirant Agent | 4.00 |

Procedure
1. Combine A and B with overhead mixing (medium agitation)
2. Slowly add C. Mix well
3. Slowly add D. Mix well
4. Slowly add E and F. Mix well
5. Premix H and I. Slowly add to the main batch
6. Slowly add G. Mix well
7. Slowly add K and J. Mix well until clear
8. Pour into appropriate containers

### Example 5

A composition of the invention in aerosol form is made by the following procedure:

**TABLE 5**

| INGREDIENT | FUNCTION | % w/w |
|---|---|---|
| A: Bentone Gel VS-5 PC | Suspending Agent | 18.50 |
| B: Cyclomethicone | Solvent | 21.20 |
| C: Dimethicone, 50 cst | Skin Conditioning | 8.50 |
| D: Isopropyl Myristate | Skin Conditioning | 1.50 |
| E: Absorbents | Absorbents | 6.00 |
| F: Fragrance | Perfume | 0.30 |
| G: Acetyl octapeptide-3 or dipeptide diaminobutyroyl benzylamide diacetate | Antiperspirant Agent | 4.00 |
| H: Isobutane | Propellant | 40.00 |

Procedure
1. Combine A, B, C and D. Mix well for about 10 min or until homogeneous.
2. Add E mix until uniform
3. Add G mix until uniform
4. Add F and mix 5 min
5. Place mixture into a suitable container and charge with H as specified.

### Example 6

The following composition according to the invention is a solid formulation.

**TABLE 6**

| INGREDIENT | FUNCTION | % w/w |
|---|---|---|
| A: Volatile Silicone | Solvent | 56.70 |
| B: Stearyl Alcohol | Surfactant | 20.00 |
| C: PPG-3 Myristyl Ether | Skin Conditioning | 5.00 |
| D: PEG-8 Distearate | Surfactant | 2.00 |
| E: Talc | Absorbent | 1.00 |
| F: Silica | Absorbent | 1.50 |
| G: Polyethylene | Film Former | 3.00 |
| H: Absorbents | Absorbents | 6.00 |
| I: Fragrance | Perfume | 0.80 |
| J: Acetyl octapeptide-3 or dipeptide diaminobutyroyl benzylamide diacetate | Antiperspirant Agent | 4.00 |

Procedure
1. Heat A to 65° C
2. Add C and D with stirring
3. Add B slowly. Maintain 65° C
4. Add E, F, G and H.
5. Cool to 60°C. Add I and J.
6. Pour into stick casings at 54°C.

### Example 7

Using the ingredients in Table 7 and the procedure below, a comparative roll-on composition containing acetyl hexapeptide-8 was prepared.

**TABLE 7**

| INGREDIENT | FUNCTION | % w/w |
|---|---|---|
| A: Aqua | Solvent | 79.51 |
| B: Steareth-2 | Surfactant | 3.00 |
| C: Steareth-21 | Surfactant | 1.00 |
| D: PPG-15 Stearyl Ether | Emollient | 4.00 |
| E: Ethylhexyglycerin; Caprylyl Glycol | Skin Conditioning Agent | 1.00 |
| F: Polyglyceryl-3 Caprylate | Emulsifying Agent | 0.30 |
| G: Citric acid | pH adjuster | 0.68 |
| H: Sodium Hydroxide | pH adjuster | 0.01 |
| I: Sodium Benzoate | Preservative | 0.50 |
| J: Acetyl Hexapeptide-8 | Antiperspirant agent | 10.00 |

Procedure:
1. Oily Phase: Combined B, C, D, E and F in an auxiliary tank. Started heating to 75-80°C.
2. Water Phase: Combined A and I in a main tank. Started heating to 75°-80°C.
3. Emulsification Step: Added oily phase over water phase (both 75-80°C). Mixed for 5 min. Homogenized for 4 min.
4. Cooling Step: Cooled the emulsion to 25° C. Added G, H and J. Mixed for 10 min.

### Example 8

Using the ingredients in Table 8 and the procedure below, a roll-on composition containing acetyl cctapeptide-3 according to the invention was prepared.

**TABLE 8**

| INGREDIENT | FUNCTION | % w/w |
|---|---|---|
| A: Aqua | Solvent | 79.51 |
| B: Steareth-2 | Surfactant | 3.00 |
| C: Steareth-21 | Surfactant | 1.00 |
| D: PPG-15 Stearyl Ether | Emollient | 4.00 |
| E: Ethylhexyglycerin; Caprylyl Glycol | Skin Conditioning Agent | 1.00 |
| F: Polyglyceryl-3 Caprylate | Emulsifying Agent | 0.30 |
| G: Citric acid | pH adjuster | 0.68 |
| H: Sodium Hydroxide | pH adjuster | 0.01 |
| I: Sodium Benzoate | Preservative | 0.50 |
| J: Acetyl Octapeptide-3 | Antiperspirant agent | 10.00 |

Procedure:
1. Oily Phase: Combined B, C, D, E and F in an auxiliary tank. Started heating to 75-80° C.
2. Water Phase: Combined A and I in a main tank. Started heating to 75°-80° C.
3. Emulsification Step: Added oily phase over water phase (both 75-80 °C). Mixed for 5 min. Homogenized for 4 min.
4. Cooling Step: Cooled the emulsion to 25° C. Add G, H and J. Mixed it for 10 min.

### Example 9

Using the ingredients in Table 9 and the procedure below, a roll-on composition containing dipeptide diaminobutyroyl benzylamide diacetate according to the invention was prepared.

**TABLE 9**

| INGREDIENT | FUNCTION | % w/w |
|---|---|---|
| A: Aqua | Solvent | 84.46 |
| B: Steareth-2 | Surfactant | 3.00 |
| C: Steareth-21 | Surfactant | 1.00 |
| D: PPG-15 Stearyl Ether | Emollient | 4.00 |
| E: Ethylhexyglycerin; Caprylyl Glycol | Skin Conditioning Agent | 1.00 |
| F: Polyglyceryl-3 Caprylate | Emulsifying Agent | 0.30 |
| G: Citric acid | pH adjuster | 0.73 |
| H: Sodium Hydroxide | pH adjuster | 0.01 |
| I: Sodium Benzoate | Preservative | 0.50 |
| J: Dipeptide Diaminobutyroyl Benzylamide Diacetate | Antiperspirant agent | 5.00 |

Procedure:
1. Oily Phase: Combined B, C, D, E and F in an auxiliary tank. Started heating to 75-80° C.
2. Water Phase: Combined A and I in a main tank. Started heating to 75-80° C.
3. Emulsification Step: Added oily phase over water phase (both 75-80° C). Mixed for 5 min. Homogenized for 4 min.
4. Cooling Step: Cooled the emulsion to 25° C. Added G, H and J. Mixed it for 10 min.

### Example 10

An *in vivo* study of the eight-hour antiperspirant efficacy of the compositions of Examples 7, 8 and 9 was performed as follows using the US FOOD AND DRUG ADMINISTRATION Guidelines for Effectiveness Testing of OTC Antiperspirant Drug Products, 2003. Example 7 was comparative, as indicated above.

Fifteen subjects were used to test each composition. Subjects were first subjected to a 21-day conditioning period, which began with a 14 day wash out period. During 14 day wash out period, the subjects used a standard bar soap and bacteriostatic deodorant supplied by the institute running the study. The subjects did not remove underarm hair. Following this, from day 15 to day 21 the subjects were instructed to use only standard soap and discontinue the bacteriostatic deodorant.

During the conditioning period, subjects were instructed and supervised by a trained technician to wash their underarms according to the following procedure: a) wash one of the underarms for 10 seconds with an aqueous solution of standard soap 2.0%; b) rinse thoroughly with running water until the soap is completely removed; c) dry the underarm with a paper towel; and d) repeat the procedure with the other underarm.

After the wash out period was completed, each subject was checked for unauthorized use of antiperspirant products during the conditioning period using a cotton swab in the underarms.

Next, a 30 day application period began. 150 cm², randomly distributed areas were marked on each subjects' underarm. Test samples were then applied to one underarm of each subject, while the other underarm was untreated as a control every day during the 30 day application period. Subjects waxed or shaved their underarms every Friday evening.

On day 30 of the 30 day application period, each subject was provided with a 100% cotton t-shirt previously washed with water and neutral soap without fragrance and again washed only with water. The subjects were instructed to wear the T-shirts for eight hours.

After eight hours, the subjects were placed in a room at 37.8° C ± 1° C and relative humidity between 30% and 40% for 80 minutes. After 40 minutes in the hot room, pads previously weighed and stored in closed polyethylene bags were removed from the bags and placed immediately onto the subjects' underarms, and then replaced every 20 minutes. Subjects ingested 200 mL of water when entering the hot room and also 40 minutes later in order to maintain the internal level of hydration, allowing the sweat to flow freely and evidencing only the effects of the formulation of the product tested. The initial 40 minute period was considered as an acclimatization period.

Used pads were collected every 20 minutes and weighed. The weights of the pads pre- and post-use were compared according to the FDA Guidelines using statistical analysis with the Wilcoxon Signed Rank test (FDA protocol) and the Student-t test paired with the unilateral hypothesis of improvement for sweating assessment. The confidence level considered in the comparative analysis was 95%. Software: XLSTAT 2013 and MINITAB 14.

The results are shown in Table 10.

**TABLE 10**

| Anticholinergic Agent | % of subjects with reduction in relation to control | Average % of sweat reduction (mg) |
|---|---|---|
| Acetyl Hexapeptide-8 | 66.7 | 8.0% |
| Acetyl Octapeptide-3 | 81.8 | 20.7 |
| Diaminobutyroyl Benzylamide Diacetate | 92.3 | 25.6 |

The compositions containing acetyl octapeptide-3 and dipeptide diaminobutyroyl benzylamide diacetate according to the invention reduced the sweat by an average of 20.7% and 25.6%, respectively, relative to the control for at least 50% of subjects (81.8% and 92.3%). This confirms antiperspirant activity. The FDA Guidelines call for a minimum of 20% sweat reduction in at least 50% volunteers. In contrast, the comparative composition containing acetyl hexapeptide-8 showed sweat reduction by an average of only 8.0% relative to the control, which did not meet success criteria.

## Claims

1. A topical antiperspirant composition comprising a cosmetically acceptable topical carrier and an anticholinergic agent selected from the group consisting of acetyl octapeptide-3 and dipeptide diaminobutyroyl benzylamide diacetate in an amount effective to inhibit or prevent perspiration, wherein said composition is substantially free of aluminum salts.

2. The composition according to claim 1 comprising about 0.01 to about 30.0 w/w% of the anticholinergic agent.

3. The composition according to claim 1 comprising about 0.1 to about 20.0 w/w% of acetyl octapeptide-3.

4. The composition according to claim 1 comprising about 0.05 to about 6.0 w/w% of dipeptide diaminobutyroyl benzylaminde diacetate.

5. The composition according to any preceding claim further comprising a water absorbing agent.
